# FASCICULE DE BREVET EUROPEEN

(11) **EP 2 760 824 B1**
(45) Date de publication et mention de la délivrance du brevet: **28.10.2015**
(21) Numéro de dépôt: 12769372.9
(22) Date de dépôt: 27.09.2012
(51) Int. Cl.: C07C 253/10, C07C 255/03, C07C 255/04

(54) **PROCEDE DE FABRICATION DE COMPOSES NITRILES A PARTIR DE COMPOSES A INSATURATION ETHYLENIQUE**
VERFAHREN ZUR HERSTELLUNG VON NITRILVERBINDUNGEN AUS ETHYLENISCH UNGESÄTTIGTEN VERBINDUNGEN
METHOD FOR PRODUCING NITRILE COMPOUNDS FROM ETHYLENICALLY UNSATURATED COMPOUNDS

(30) Priorité: 30.09.2011 FR 1102976
(43) Date de publication de la demande: 06.08.2014
(73) Titulaire: Invista Technologies S.à.r.l., 9000 St. Gallen (CH)
(72) Inventeur: MASTROIANNI, Sergio, 69006 Lyon (FR); PRINGLE, Paul, Bristol BS8 3DG (GB); HOPEWELL, Jonathan, Wakefield WF4 3JH (GB); GARLAND, Michael, Bradford-On-Avon Wiltshire BA15 1UT (GB)
(74) Mandataire: Carpmaels & Ransford LLP
(86) Numéro de dépôt international: PCT/EP2012/069027
(87) Numéro de publication internationale: WO 2013/045524

(56) Documents cités:
- WO-A1-2010/145960
- M. FILD ET AL: "Phosphorus-fluorine chemistry. Part XXIII. t-Butyl-fluorophosphines and -fluorophosphoranes and their derivatives", JOURNAL OF THE CHEMICAL SOCIETY A: INORGANIC, PHYSICAL, THEORETICAL, 1 janvier 1970 (1970-01-01), page 2359, XP055035380, ISSN: 0022-4944, DOI: 10.1039/j19700002359 cité dans la demande
- NATALIE FEY ET AL: "Stable Fluorophosphines: Predicted and Realized Ligands for Catalysis", ANGEWANDTE CHEMIE INTERNATIONAL EDITION, vol. 51, no. 1, 2 janvier 2012 (2012-01-02), pages 118-122, XP055035431, ISSN: 1433-7851, DOI: 10.1002/anie.201105954

## Description

La présente invention concerne un procédé d'hydrocyanation de composés organiques à insaturation éthylénique en composés comprenant au moins une fonction nitrile.

Elle se rapporte plus particulièrement à l'hydrocyanation de dioléfines telles que le butadiène ou d'oléfines substituées telles que des alcènes-nitriles comme les pentènenitriles.

La réaction d'hydrocyanation est, par exemple, décrite dans le brevet français n° 1 599 764 qui concerne un procédé de préparation de nitriles par addition d'acide cyanhydrique sur des composés organiques ayant au moins une double liaison éthylénique, en présence d'un catalyseur comprenant du nickel et un ligand organophosphorés, un triarylphosphite. Cette réaction peut être conduite en présence ou non d'un solvant.

Quand un solvant est utilisé, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

De nombreux autres systèmes catalytiques ont été proposés comprenant généralement des composés organophosphorés appartenant à la famille des phosphites, phosphonites, phosphinites et phosphines. Ces composés organophosphorés peuvent comprendre un atome de phosphore par molécule et sont qualifiés de ligands monodentates, ou plusieurs atomes de phosphore par molécules, ils sont alors appelés ligands pluridentates. Plus particulièrement de nombreux ligands contenant deux atomes de phosphore par molécule (ligand bidentate) ont été décrits dans de nombreux brevets.

WO 2010/145960 A1 divulgue un procédé d'hydrocyanation de composés organiques à insaturation éthylénique en composés comprenant au moins une fonction nitrile dans lequel des ligands organophosphorés fluorés de type R₅O(R₆O)PF ou R₇PF (dans lequel R₇ et P forment un groupe cyclique) sont utilisés.

Toutefois, la recherche de nouveaux systèmes catalytiques plus performants tant en activité catalytique qu'en stabilité est toujours entreprise pour améliorer l'économie générale du procédé.

Un des buts de la présente invention est de proposer une nouvelle famille de ligands qui permet d'obtenir avec les métaux de transition des systèmes catalytiques présentant une bonne activité catalytique notamment dans les réactions d'hydrocyanation.

A cet effet, la présente invention propose un procédé d'hydrocyanation d'un composé hydrocarboné comprenant au moins une insaturation éthylénique par réaction en milieu liquide avec le cyanure d'hydrogène en présence d'un catalyseur comprenant un élément métallique choisi parmi les métaux de transition et un ligand organophosphoré, le ligand organophosphoré comprenant un composé répondant à la formule générale (I): 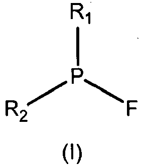 dans laquelle :
R₁ et R₂, identiques ou différents, représentent un radical alkyl linéaire ou ramifié ayant de 1 à 12 atomes de carbone pouvant contenir des hétéroatomes, ou un radical comprenant un radical aromatique ou cycloaliphatique substitué ou non pouvant comprendre des hétéroatomes
la liaison covalente entre P et R₁ d'une part, et celle entre P et R₂ d'autre part, sont des liaisons P-C

Avantageusement R₁ et R₂ représentent un radical alkyl linéaire ou ramifié ayant de 1 à 12 atomes de carbone pouvant contenir des hétéroatomes.

Comme composé de formule générale (I) convenable pour le procédé de l'invention, on peut citer par exemple ^{t}Bu₂PF.

Les composés de formule générale (I) peuvent être préparés selon toute méthode connue de l'homme du métier. Ils peuvent par exemple être obtenus par réaction entre les composés R₁R₂PCl ou R₁R₂PI, R₁ et R₂ étant les radicaux de la formule générale (I), avec un sel fluoré, par exemple CsF. Une telle synthèse, pour préparer le composé ^{t}Bu₂PF, est par exemple décrite dans la publication Schmutzler et al. *J. Chem. Soc.* (A), 1970, 2359-2364..

Selon l'invention, les composés organophosphorés de formules (I) sont utilisés pour la fabrication de système catalytique par association avec un élément métallique pour former un complexe. Globalement, la composition de ces systèmes catalytiques peut être représentée par la formule générale (II) (cette formule ne correspond pas à la structure des composés et complexes présents dans le système catalytique):

M[L_{f}]ₜ (II)

dans laquelle:
M est un métal de transition
L_{f} représente au moins un ligand organophosphoré de formule (I)
t représente un nombre compris entre 1 et 10 (bornes incluses)

Les métaux M qui peuvent être complexés sont de manière générale tous les métaux de transition des groupes 1 b, 2b, 3b, 4b, 5b, 6b, 7b et 8 de la Classification périodique des éléments, telle que publiée dans "Handbook of Chemistry and Physics, 51st Edition (1970-1971)" de The Chemical Rubber Company.

Parmi ces métaux, on peut citer plus particulièrement les métaux pouvant être utilisés comme catalyseurs dans les réactions d'hydrocyanation. Ainsi on peut mentionner à titre d'exemples non limitatifs, le nickel, le cobalt, le fer, le ruthénium, le rhodium, le palladium, l'osmium, l'iridium, le platine, le cuivre, l'argent, l'or, le zinc, le cadmium, le mercure. Le nickel est l'élément préféré pour l'hydrocyanation des oléfines et des nitriles insaturés.

La préparation des systèmes catalytiques comprenant des composés de formule générale (I) peut être effectuée en mettant en contact une solution d'un composé du métal choisi, par exemple le nickel, avec une solution du composé organophosphoré de l'invention.

Le composé du métal peut être dissous dans un solvant. Le métal peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le rhodium est au degré d'oxydation (I), le ruthénium au degré d'oxydation (II), le platine au degré d'oxydation (0), le palladium au degré d'oxydation (0), l'osmium au degré d'oxydation (II), l'iridium au degré d'oxydation (I), le nickel au degré d'oxydation (0).

Si lors de la préparation du complexe organométallique, le métal est mis en oeuvre à un degré d'oxydation plus élevé, il pourra être réduit in situ.

Parmi les composés de métaux M utilisables pour la préparation des complexes organométalliques, notamment quand le métal est le nickel, on peut citer, à titre d'exemples non limitatifs, les composés suivants du nickel :
- les composés dans lesquels le nickel est au degré d'oxydation zéro comme le tétracyanonickelate de potassium K₄ [Ni(CN)₄], le bis (acrylonitrile) nickel zéro, le bis (cyclooctadiène-1,5) nickel (appelé également Ni(cod)₂) et les dérivés contenant des ligands comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.

Quand on utilise un composé du fer, les mêmes réducteurs conviennent. Dans le cas du palladium, les réducteurs peuvent être, en outre, des éléments du milieu réactionnel (solvant, oléfine).

Les composés organiques comportant au moins une double liaison éthylénique plus particulièrement mis en oeuvre dans le présent procédé sont des dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3-nitrile, le pentène-4-nitrile et également les monooléfines comme le styrène, le méthyl-styrène, le vinyl-naphtalène, le cyclohexène, le méthyl-cyclohexène ainsi que les mélanges de plusieurs de ces composés.

Les pentène-nitriles peuvent contenir, en plus du pentène-3-nitrile et du pentène-4-nitrile, des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentène-nitriles linéaires des quantités non négligeables de méthyl-2-butène-3-nitrile et de méthyl-2-butène-2-nitrile.

Le système catalytique utilisé pour l'hydrocyanation selon le procédé de l'invention peut être préparé avant son introduction dans la zone de réaction, par exemple par addition au composé de formule (I) seul ou dissous dans un solvant, la quantité appropriée de composé du métal de transition choisi et éventuellement du réducteur. Il est également possible de préparer le système catalytique "in situ" par simple addition du composé de formule (I) et du composé du métal de transition dans le milieu réactionnel d'hydrocyanation avant ou après l'addition du composé à hydrocyaner.

La quantité de composé du nickel ou d'un autre métal de transition utilisée est choisie pour obtenir une concentration en mole de métal de transition par mole de composés organiques à hydrocyaner ou isomériser comprise entre 10⁻⁴ et 1, et de préférence entre 0,005 et 0,5 mole de nickel ou de l'autre métal de transition mis en oeuvre.

La quantité de composé de formule (I) utilisée pour former le catalyseur est choisie de telle sorte que le nombre de moles de ce composé rapporté à 1 mole de métal de transition soit de 0,5 à 100 et de préférence de 0,5 à 50.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation. A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C. Elle peut être réalisée en milieu monophasique.

Le procédé d'hydrocyanation de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu tel que le procédé Andrussov consistant à faire réagir le méthane avec l'ammoniac et de l'air.

Le cyanure d'hydrogène exempt d'eau est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le composé de formule I, le composé de métal de transition (le nickel), les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés et séparés, par exemple, par distillation.

Avantageusement, la synthèse de dinitriles tels que l'adiponitrile à partir de dioléfines (butadiène) est obtenue en deux étapes successives. La première étape consiste à hydrocyaner une double liaison de la dioléfine pour obtenir un mononitrile insaturé. La seconde étape consiste à hydrocyaner l'insaturation du mononitrile pour obtenir le ou les dinitriles correspondants. Ces deux étapes sont généralement mises en oeuvre avec un système catalytique comprenant un complexe organométallique de même nature. Toutefois, les rapports composé organophosphoré/ élément métallique et concentration du catalyseur peuvent être différents. En outre, il est préférable d'associer au système catalytique un cocatalyseur ou promoteur dans la seconde étape. Ce cocatalyseur ou promoteur est généralement un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, Vlb, VIIb et VIII de la Classification périodique des éléments. Ces composés sont le plus souvent des sels, notamment des halogénures, comme chlorures ou bromures, sulfates, sulfonates, halogénosulfonates, perhalogénoalkyl sulfonates, notamment fluoroalkylsulfonates ou perfluoroalkylsulfonates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés organométalliques comme le triphénylborane, l'isopropylate de titane ou les composés décrits dans les demandes de brevet français FR 2926816 et FR 2937321.

On peut bien entendu mettre en oeuvre des mélanges de plusieurs acides de Lewis, comme cela est décrit par exemple dans la demande de brevet français FR 2941455.

Parmi les acides de Lewis, on préfère tout particulièrement le chlorure de zinc, le bromure de zinc, le chlorure stanneux, le bromure stanneux, le triphénylborane et les mélanges chlorure de zinc/chlorure stanneux.

Le cocatalyseur acide de Lewis mis en oeuvre représente généralement de 0,01 à 50 moles par mole de composé de métal de transition, plus particulièrement de composé du nickel, et de préférence de 1 à 10 mole par mole.

Les mononitriles insaturés mis en oeuvre dans cette deuxième étape sont avantageusement des pentène-nitriles linéaires comme le pentène-3-nitrile, le pentène-4-nitrile et leurs mélanges.

Ces pentène-nitriles peuvent contenir des quantités, généralement minoritaires, d'autres composés, comme le méthyl-2-butène-3-nitrile, le méthyl-2-butène-2-nitrile, le pentène-2-nitrile.

La solution catalytique utilisée pour l'hydrocyanation en présence d'acide de Lewis peut être préparée avant son introduction dans la zone de réaction, par exemple par addition au composé de formule (I), de la quantité appropriée de composé du métal de transition choisi, de l'acide de Lewis et éventuellement du réducteur. Il est également possible de préparer la solution catalytique "in situ" par simple addition de ces divers constituants dans le milieu réactionnel.

Il est également possible dans les conditions du procédé d'hydrocyanation de la présente invention, et notamment en opérant en présence du système catalytique décrit précédemment comportant un composé de formule (I) et au moins un composé d'un métal de transition, de réaliser, en absence de cyanure d'hydrogène, l'isomérisation du méthyl-2-butène-3-nitrile en pentènenitriles, et plus généralement des nitriles insaturés ramifiés en nitriles insaturés linéaires.

Le méthyl-2-butène-3-nitrile soumis à l'isomérisation selon l'invention peut être mis en oeuvre seul ou en mélange avec d'autres composés. Ainsi on peut engager du méthyl-2-butène-3-nitrile en mélange avec du méthyl-2-butène-2 nitrile, du pentène-4-nitrile, du pentène-3-nitrile, du pentène-2-nitrile, du butadiène.

Il est particulièrement intéressant de traiter le mélange réactionnel provenant de l'hydrocyanation du butadiène par HCN en présence d'au moins un composé de formule (I) et d'au moins un composé d'un métal de transition, plus préférentiellement d'un composé du nickel au degré d'oxydation 0, tel que défini précédemment. Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température comprise entre 10°C et 200°C et de préférence entre 60°C et 140°C.

Dans le cas préféré d'une isomérisation suivant immédiatement la réaction d'hydrocyanation du butadiène, il sera avantageux d'opérer à la température à laquelle l'hydrocyanation a été conduite ou légèrement supérieure.

Comme pour le procédé d'hydrocyanation de composés à insaturation éthylénique, le système catalytique utilisé pour l'isomérisation peut être préparé avant son introduction dans la zone de réaction, par exemple par mélange du composé de formule (I), de la quantité appropriée de composé du métal de transition choisi et éventuellement du réducteur. Il est également possible de préparer le système catalytique "in situ" par simple addition de ces divers constituants dans le milieu réactionnel. La quantité de composé du métal de transition et plus particulièrement du nickel utilisée, ainsi que la quantité de composé de formule (I) sont les mêmes que pour la réaction d'hydrocyanation.

Bien que la réaction d'isomérisation soit conduite généralement sans solvant, il peut être avantageux de rajouter un solvant organique inerte qui pourra être utilisé comme solvant d'extraction, ultérieurement. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme à l'invention pour les étapes de formation des nitriles insaturés et l'étape d'isomérisation ci-dessus, la réaction d'hydrocyanation des nitriles insaturés en dinitriles pouvant être mise en oeuvre avec un système catalytique conforme à l'invention ou tout autre système catalytique déjà connu pour cette réaction.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mise en oeuvre avec un système catalytique conforme à l'invention.

D'autres détails, avantages de l'invention seront illustrés par les exemples donnés ci-dessous uniquement à titre indicatif, sans caractère limitatif.

### EXEMPLES

### Abréviations utilisées

- Cod: cyclooctadiène
- Ni(Cod)₂ : bis(1,5-cyclooctadiène)nickel
- 3PN: 3-pentènenitrile
- AdN: Adiponitrile - ESN: éthylsuccinonitrile
- MGN: méthylglutaronitrile
- DN : composés dinitriles (AdN, MGN ou ESN)
- TIBAO: tetraisobutyldialuminoxane
- RR(DN): rendement réel de dinitriles correspondant au rapport du nombre de moles formées de dinitriles sur le nombre de moles de 3PN engagé
- Linéarité (L): rapport du nombre de moles d'AdN formées sur le nombre de moles de dinitriles formées (somme des moles d'AdN, ESN et MGN)

Les composés suivants: 3PN, Ni(Cod)₂, ZnCl₂, TIBAO et Ph₂BOBPh₂ (anhydride diphénylborinique) sont disponibles commercialement.

### Exemple 1 : Préparation du ligand ^{t}Su₂PF

### Ligand

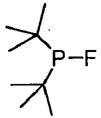

La synthèse de ^{t}Bu₂PF a été publiée par Schmutzler et al. J. Chem. Soc. (A), 1970, 2359-2364.

^{t}Bu₂PCl (2,20 g, 12,2 mmol) liquide est ajouté lentement (15 min) à une suspension de CsF (4,63 g, 30,5 mmol) dans MeCN (10 cm³) et le mélange est agité à température ambiante pendant 2 h. Le mélange réactionnel est ensuite filtré et la solution incolore obtenue est séparée par distillation fractionnée. La première fraction contient uniquement de l'acétonitrile, alors que la deuxième (point d'ébullition 110-112 °C) et troisième fraction (129-131°C) sont similaires selon les analyses RMN ³¹P et ¹H et ont été réunies (volume total 13,5 cm³). Elles contiennent uniquement de l'acétonitrile et du ^{t}Bu₂PF
³¹P{¹H} NMR (*in situ*): 216,1 (d, J_{PF} = 862 Hz),
¹H NMR (CDCl₃) 1,95 (3H, s, MeCN), 1,12 (6H, dd, J_{HP} = 11,50 Hz, J_{HF} = 1,65 Hz)
Le rapport molaire ^{t}Bu₂PF:MeCN est de 1:45 dans la solution

### Exemples 2 à 4 : Hydrocyanation du 3-PN en AdN

Le mode opératoire général utilisé est le suivant

Sous atmosphère d'argon, dans un tube de verre type Shott de 60 ml équipé d'un bouchon-septum, sont chargés successivement :
- le ligand (1 mmol, 2 équivalents en P)
- 1,24 g (15 mmol, 30 équivalents) de 3PN anhydre
- 138 mg (0,5 mmol, 1 équivalent) de Ni(cod)₂
- acide de Lewis (voir tableau 1 pour la nature et la quantité)

Le mélange est porté sous agitation à 70°C. La cyanhydrine de l'acétone est injectée dans le milieu réactionnel par un pousse-seringue à un débit de 0,45 ml par heure. Après 3 heures d'injection le poussé seringue est arrêté. Le mélange est refroidi à température ambiante, dilué à l'acétone et analysé par chromatographie en phase gazeuse.

Les résultats sont regroupés dans le tableau suivant :

**Tableau**

| Exemple | Ligand | Acide de Lewis | Acide de Lewis/Ni (molaire) | Linéarité | RR (DN) |
|---|---|---|---|---|---|
| 2 | ^{t}Bu₂PF | ZnCl₂ | 1 | 50 | 4,3 |
| 3 | ^{t}Bu₂PF | TIBAO | 0,5 | 46,8 | 10 |
| 4 | ^{t}Bu₂PF | Ph₂BOBPh₂ | 0,5 | 52,1 | 6 |

## Revendications

1. Procédé d'hydrocyanation d'un composé hydrocarboné comprenant au moins une insaturation éthylénique par réaction en milieu liquide avec le cyanure d'hydrogène en présence d'un catalyseur comprenant un élément métallique choisi parmi les métaux de transition et un ligand organophosphoré **caractérisé en ce que** le ligand organophosphoré comprend un composé répondant à la formule générale (I): 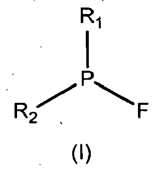 dans laquelle :
R₁ et R₂, identiques ou différents, représentent un radical alkyl linéaire ou ramifié ayant de 1 à 12 atomes de carbone pouvant contenir des hétéroatomes, ou un radical comprenant un radical aromatique ou cycloaliphatique substitué ou non pouvant comprendre des hétéroatomes
la liaison covalente entre P et R₁ d'une part, et celle entre P et R₂ d'autre part, sont des liaisons P-C.

2. Procédé selon la revendication 1, **caractérisé en ce que** R₁ et R₂ représentent un radical alkyl linéaire ou ramifié ayant de 1 à 12 atomes de carbone pouvant contenir des hétéroatomes

3. Procédé selon la revendication 1 ou 2, **caractérisé en ce que** le composé de formule (I) est ^{t}Bu₂PF.

4. Procédé selon l'une des revendications précédentes, caractérisé en ce l'élément métallique est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure

5. Procédé selon l'une des revendications précédentes, **caractérisé en ce que** la composition du système catalytique est exprimée par la formule générale (II):
M[L_{f}]ₜ (II)
dans laquelle:
M est un métal de transition
L_{f} représente au moins un ligand organophosphoré de formule (I)
t représente un nombre compris entre 1 et 10 (bornes incluses)

6. Procédé selon l'une des revendications précédentes, **caractérisé en ce que** les composés organiques comportant au moins une insaturation éthylénique sont choisis parmi les dioléfines comme le butadiène, l'isoprène, l'hexadiène-1,5, le cyclooctadiène-1,5, les nitriles aliphatiques à insaturation éthylénique, particulièrement les pentènenitriles linéaires comme le pentène-3-nitrile, le pentène-4-nitrile, les monooléfines comme le styrène, le méthyl-styrène, le vinyl-naphtalène, le cyclohexène, le méthyl-cyclohexène ainsi que les mélanges de plusieurs de ces composés.

7. Procédé selon l'une des revendications précédentes, **caractérisé en ce que** la quantité de composé du nickel ou d'un autre métal de transition est choisie de telle sorte qu'il y ait par mole de composé organique à hydrocyaner ou isomériser entre 10⁻⁴ et 1 mole de nickel ou de l'autre métal de transition mis en oeuvre et **en ce que** la quantité de composé organosphosphoré utilisée est choisie de telle sorte que le nombre de moles de ces composés rapporté à 1 mole de métal de transition soit de 0,5 à 100.

8. Procédé selon l'une des revendications précédentes, **caractérisé en ce qu'**il s'agit d'un procédé d'hydrocyanation en dinitriles, que les composés à insaturation éthyléniques sont des composés nitriles à insturation éthyléniques et que l'on opère en présence d'un système catalytique comprenant au moins un composé d'un métal de transition, au moins un composé de formule (I) et un cocatalyseur consistant en au moins un acide de Lewis.

9. Procédé selon la revendication 8, **caractérisé en ce que** les composés nitriles à insaturation éthylénique sont choisis parmi les nitriles aliphatiques à insaturation éthylénique comprenant les pentènenitriles linéaires comme le pentène-3-nitrile, le pentène-4-nitrile et leurs mélanges.

10. Procédé selon l'une des revendications 8 ou 9, **caractérisé en ce que** l'acide de Lewis mis en oeuvre comme cocatalyseur est choisi parmi les composés des éléments des groupes Ib, IIb, IIIa, IIIb, IVa, IVb, Va, Vb, Vlb, VIIb et VIII de la Classification périodique des éléments.

11. Procédé selon l'une des revendications 8 à 10, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium et leurs mélanges, les composés organométalliques.

12. Procédé selon l'une des revendications précédentes, **caractérisé en ce que** l'on réalise en absence de cyanure d'hydrogène l'isomérisation en pentènenitriles, du méthyl-2-butène-3-nitrile présent dans le mélange réactionnel provenant de l'hydrocyanation du butadiène, en opérant en présence d'un catalyseur comportant au moins un composé de formule (I) et au moins un composé d'un métal de transition.

## Patentansprüche

1. Verfahren zur Hydrocyanierung einer Kohlenwasserstoffverbindung mit mindestens einer ethylenischen Ungesättigtheit durch Umsetzung mit Cyanwasserstoff in flüssigem Medium in Gegenwart eines Katalysators, der ein aus den Übergangsmetallen ausgewähltes Metallelement und einen Organophosphorliganden umfasst, **dadurch gekennzeichnet, dass** der Organophosphorligand eine Verbindung umfasst, die der allgemeinen Formel (I) entspricht: 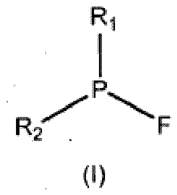 worin:
R₁ und R₂ gleich oder verschieden sind und für einen linearen oder verzweigten Alkylrest mit 1 bis 12 Kohlenstoffatomen, der Heteroatome enthalten kann, oder einen Rest, der einen gegebenenfalls substituierten aromatischen oder cycloaliphatischen Rest umfasst, der Heteroatome umfassen kann, stehen,
es sich bei der kovalenten Bindung zwischen P und R₁ einerseits und bei der kovalenten Bindung zwischen P und R₂ andererseits um P-C-Bindungen handelt.

2. Verfahren nach Anspruch 1, **dadurch gekennzeichnet, dass** R₁ und R₂ für einen linearen oder verzweigten Alkylrest mit 1 bis 12 Kohlenstoffatomen, der Heteroatome enthalten kann, stehen.

3. Verfahren nach Anspruch 1 oder 2, **dadurch gekennzeichnet, dass** es sich bei der Verbindung der Formel (I) um ^{t}Bu₂PF handelt.

4. Verfahren nach einem der vorhergehenden Ansprüche, **dadurch gekennzeichnet, dass** man das Metallelement aus der Gruppe bestehend aus Nickel, Cobalt, Eisen, Ruthenium, Rhodium, Palladium, Osmium, Iridium, Platin, Kupfer, Silber, Gold, Zink, Cadmium und Quecksilber auswählt.

5. Verfahren nach einem der vorhergehenden Ansprüche, **dadurch gekennzeichnet, dass** die Zusammensetzung des Katalysatorsystems der allgemeinen Formel (II) entspricht:
M[L_{f}]ₜ (II)
worin:
M für ein Übergangsmetall steht,
L_{f} für mindestens einen Organophosphorliganden der Formel (I) steht,
t für eine Zahl zwischen 1 und 10 (einschließlich der Grenzwerte) steht.

6. Verfahren nach einem der vorhergehenden Ansprüche, **dadurch gekennzeichnet, dass** man die organischen Verbindungen mit mindestens einer ethylenischen Ungesättigtheit aus Diolefinen wie Butadien, Isopren, 1,5-Hexadien, 1,5-Cyclooctadien, aliphatischen Nitrilen mit ethylenischer Ungesättigtheit, insbesondere linearen Pentennitrilen wie 3-Pentennitril und 4-Pentennitril, Monoolefinen wie Styrol, Methylstyrol, Vinylnaphthalin, Cyclohexen, Methylcyclohexen und Mischungen mehrerer dieser Verbindungen auswählt.

7. Verfahren nach einem der vorhergehenden Ansprüche, **dadurch gekennzeichnet, dass** man die verwendete Menge der Verbindung von Nickel oder einem anderen Übergangsmetall so wählt, dass pro Mol zu hydrocyanierende oder isomerisierende organische Verbindung zwischen 10⁻⁴ und 1 mol verwendetes Nickel oder anderes Übergangsmetall vorliegen, und die verwendete Menge der Organophosphorverbindung so wählt, dass die Zahl der Mole dieser Verbindungen, bezogen auf 1 mol Übergangsmetall, 0,5 bis 100 beträgt.

8. Verfahren nach einem der vorhergehenden Ansprüche, **dadurch gekennzeichnet, dass** es sich um ein Hydrocyanierungsverfahren zu Dinitrilen handelt, dass es sich bei den Verbindungen mit ethylenischer Ungesättigtheit um Nitrilverbindungen mit ethylenischer Ungesättigtheit handelt und dass man in Gegenwart eines Katalysatorsystems, das mindestens eine Übergangsmetallverbindung, mindestens eine Verbindung der Formel (I) und einen Cokatalysator, der mindestens aus einer Lewis-Säure besteht, umfasst, arbeitet.

9. Verfahren nach Anspruch 8, **dadurch gekennzeichnet, dass** man die Nitrilverbindungen mit ethylenischer Ungesättigtheit aus aliphatischen Nitrilen mit ethylenischer Ungesättigtheit, die lineare Pentennitrile wie 3-Pentennitril, 4-Pentennitril und Mischungen davon umfassen, auswählt.

10. Verfahren nach Anspruch 8 oder 9, **dadurch gekennzeichnet, dass** man die als Cokatalysator verwendete Lewis-Säure aus den Verbindungen von Elementen der Gruppen Ib, IIb, IIIa, IIIb, IVa, IVb, Va, Vb, VIb, VIIb und VIII des Periodensystems der Elemente auswählt.

11. Verfahren nach einem der Ansprüche 8 bis 10, **dadurch gekennzeichnet, dass** man die Lewis-Säure aus Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumtrifluormethylsulfonat, den Chloriden oder Bromiden der Seltenerdmetalle wie Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid und Mischungen davon und Organometallverbindungen auswählt.

12. Verfahren nach einem der vorhergehenden Ansprüche, **dadurch gekennzeichnet, dass** man das in der Reaktionsmischung aus der Hydrocyanierung von Butadien vorliegende 2-Methyl-3-butennitril in Abwesenheit von Cyanwasserstoff und in Gegenwart eines Katalysators, der mindestens eine Verbindung der Formel (I) und mindestens eine Übergangsmetallverbindung enthält, zu Pentennitrilen isomerisiert.

## Claims

1. Process for the hydrocyanation of a hydrocarbon compound comprising at least one ethylenic unsaturation by reaction, in a liquid medium, with hydrogen cyanide in the presence of a catalyst comprising a metal element chosen from transition metals and an organophosphorus ligand, **characterized in that** the organophosphorus ligand comprises a compound corresponding to the general formula (I): 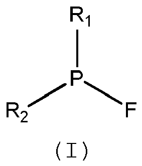 in which:
R₁ and R₂, which are identical or different, represent a linear or branched alkyl radical having from 1 to 12 carbon atoms which can comprise heteroatoms, or a radical comprising a substituted or unsubstituted aromatic or cycloaliphatic radical which can comprise heteroatoms,
the covalent bond between P and R₁, on the one hand, and that between P and R₂, on the other hand, are P-C bonds.

2. Process according to Claim 1, **characterized in that** R₁ and R₂ represent a linear or branched alkyl radical having from 1 to 12 carbon atoms which can comprise heteroatoms.

3. Process according to Claim 1 or 2, **characterized in that** the compound of formula (I) is ^{t}Bu₂PF.

4. Process according to one of the preceding claims, **characterized in that** the metal element is selected from the group consisting of nickel, cobalt, iron, ruthenium, rhodium, palladium, osmium, iridium, platinum, copper, silver, gold, zinc, cadmium and mercury.

5. Process according to one of the preceding claims, **characterized in that** the composition of the catalytic system is expressed by the general formula (II):
M [L_{f}]ₜ (II)
in which:
M is a transition metal,
L_{f} represents at least one organophosphorus ligand of formula (I),
t represents a number between 1 and 10 (limits included).

6. Process according to one of the preceding claims, **characterized in that** the organic compounds comprising at least one ethylenic unsaturation are chosen from diolefins, such as butadiene, isoprene, 1,5-hexadiene or 1,5-cyclooctadiene, ethylenically unsaturated aliphatic nitriles, particularly linear pentenenitriles, such as 3-pentenenitrile or 4-pentenenitrile, monoolefins, such as styrene, methylstyrene, vinylnaphthalene, cyclohexene or methylcyclohexene, and the mixtures of several of these compounds.

7. Process according to one of the preceding claims, **characterized in that** the amount of compound of nickel or of another transition metal is chosen so that there is, per mole of organic compound to be hydrocyanated or isomerized, between 10⁻⁴ and 1 mol of nickel or of the other transition metal employed and **in that** the amount of organophosphorus compound used is chosen so that the number of moles of these compounds, with respect to 1 mol of transition metal, is from 0.5 to 100.

8. Process according to one of the preceding claims, **characterized in that** it is a process of hydrocyanation to give dinitriles, that the ethylenically unsaturated compounds are ethylenically unsaturated nitrile compounds and that the operation is carried out in the presence of a catalytic system comprising at least one compound of a transition metal, at least one compound of formula (I) and a cocatalyst consisting of at least one Lewis acid.

9. Process according to Claim 8, **characterized in that** the ethylenically unsaturated nitrile compounds are chosen from ethylenically unsaturated aliphatic nitriles comprising linear pentenenitriles, such as 3-pentenenitrile, 4-pentenenitrile and their mixtures.

10. Process according to either of Claims 8 and 9, **characterized in that** the Lewis acid employed as cocatalyst is chosen from the compounds of the elements of Groups Ib, IIb, IIIa, IIIb, IVa, IVb, Va, Vb, VIb, VIIb and VIII of the Periodic Table of the Elements.

11. Process according to one of Claims 8 to 10, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulfate, stannous tartrate, indium trifluoromethylsulfonate, chlorides or bromides of the rare earth metal elements, such as lanthanum, cerium, praseodymium, neodymium, samarium, europium, gadolinium, terbium, dysprosium, hafnium, erbium, thallium, ytterbium and lutetium, cobalt chloride, ferrous chloride, yttrium chloride and their mixtures, or organometallic compounds.

12. Process according to one of the preceding claims, **characterized in that** the isomerization to give pentenenitriles of 2-methyl-3-butenenitrile present in the reaction mixture originating from the hydrocyanation of butadiene is carried out in the absence of hydrogen cyanide, the operation being carried out in the presence of a catalyst comprising at least one compound of formula (I) and at least one compound of a transition metal.
